# EUROPÄISCHE PATENTSCHRIFT

(11) **EP 1 125 943 B1**
(45) Veröffentlichungstag und Bekanntmachung des Hinweises auf die Patenterteilung: **06.10.2004**
(21) Anmeldenummer: 00103101.2
(22) Anmeldetag: 16.02.2000
(51) Int. Cl.: C07H 1/08, C12N 15/10

(54) **Verfahren zur Aufreinigung von Nukleinsäuren sowie Anionenaustauscher zur Durchführung dieses Verfahrens**
Process for the purification of nucleic acids and anion exchanger to carry out the process
Procédé de purification d'acides nucléiques et échangeur anionique pour la mise en oeuvre du procédé

(43) Veröffentlichungstag der Anmeldung: 22.08.2001
(73) Patentinhaber: Macherey, Nagel GmbH & Co. Handelsgesellschaft, 52355 Düren (DE)
(72) Erfinder: Zinn, Thomas, Dr., 52355 Düren (DE); Sieber, Manfred, Dr., 52385 Nideggen (DE); Zeidler, Robert, Dr., 52372 Kreuzau (DE)
(74) Vertreter: Paul, Dieter-Alfred, Dipl.-Ing.

(56) Entgegenhaltungen:
- EP-A- 0 289 129
- EP-A- 0 308 239
- WO-A-95/21179
- WO-A-97/29113
- WO-A-99/00168
- WO-A-99/63076
- US-A- 4 412 985

## Beschreibung

Die Erfindung betrifft ein Verfahren zur Aufreinigung von Nukleinsäuren aus Ausgangsmaterialien, bei dem die Nukleinsäuren, enthalten in einer biologischen Lösung, an einem Anionenaustauscher aufgereinigt werden und bei dem mit Hilfe eines nichtionischen Tensids Endotoxine teilweise oder ganz entfernt werden. Die Erfindung bezieht sich ferner auf eine Chromatographiesäule zur Durchführung dieses Verfahrens, die einen von Abschlußfiltern eingeschlossenen Anionenaustauscher enthält.

Zur therapeutischen Behandlung bestimmter Krankheiten wird zunehmend die Gentherapie angewandt. Hierzu werden aus biologischem Ausgangsmaterial, z. B. gentechnisch veränderte Bakterien, Präparate gewonnen, die Makromoleküle wie Nukleinsäuren oder Proteine als Wirkstoffe enthalten. Die Präparate werden dann dem menschlichen Organismus zugeführt.

Wesentlich für den Erfolg der Gentherapie ist es, daß das Präparat frei von toxischen Substanzen ist. Da die entsprechenden Präparate in der Regel aus Bakterien wie dem gram-negativen Enterobakterium E. coli isoliert werden und diese Bakterien als Zellwandbestandteile Lypopolysaccharide (LPS), welche auch als Endotoxine bezeichnet werden, enthalten, kommt der Entfernung solcher Endotoxine große Bedeutung zu. Intravenös zugeführte Präparate führen nämlich zu Fieber, entzündlichen Reaktionen oder zu irreversiblen Schockzuständen, auch wenn sie nur geringe Konzentrationen von Endotoxinen enthalten.

Zur Entfernung der Endotoxine sind verschiedene Verfahren bekannt. In einer Reihe von Verfahren werden hierzu nichtionische Tenside, beispielsweise das Tensid Triton X-114® der Firma Rohm & Haas, eingesetzt, und zwar im Zusammenhang mit dem für die Aufreinigung und Isolierung der Makromoleküle angewandten Verfahrens. Bei diesen Verfahren wird das die Makromoleküle enthaltende biologische Ausgangsmaterial nach einem gängigen Verfahren aufgeschlossen. Der Zellaufschluß kann dabei durch die sogenannte alkalische Lyse, durch die Einwirkung hoher Temperaturen (boiling lysis), durch das Einwirken mechanischer Kräfte (Kompression/Expansion, french press), oder durch das Einwirken von die Zellmembran zerstörenden Enzymen oder Detergenzien erfolgen. Das so erhaltene Zell-Lysat wird anschließend von Zelltrümmern durch mechanische Verfahren, beispielsweise Filtration oder zentrifugation, befreit.

In einem nachfolgenden Schritt wird das so erhaltene Lysat - auch "cleared lysate" genannt - chromatographisch aufgereinigt. Hierzu werden die im Lysat enthaltenen Makromoleküle an ein Anionenaustauscher mit Anionenaustauscherfunktion gebunden. Dabei wird im Stand der Technik eine Kartusche verwendet, in das Anionenaustauscher eingesetzt ist. Bevorzugt kann es sich dabei um ein poröses oder nichtporöses, mit Anionenaustauscherfunktion modifizierte, anorganisches oder organisches Sorbens handeln. Zur Entfernung unerwünschter Substanzen wird ein Waschpuffer hinzugegeben. Anschließend erfolgt die Elution der Makromoleküle durch Zugabe eines Elutionspuffers. Die eluierten Makromoleküle werden präzipitiert und stehen dann für weitere Anwendungen zur Verfügung.

Zur Entfernung der Endotoxine schlagen Aida et al. (Journal of Immunological Methods, 132 (1990), 191-195) und Manthorpe et al. (Human Gene Therapy 4 (1993), 419-431) vor, die Lösung mit den Makromolekülen nach deren chromatographischer Isolierung und Aufreinigung mit dem Tensid Triton X-114® zu versetzen und die Lösung zu inkubieren. Anschließend werden die an das Tensid gebundenen Endotoxine durch Phasenseparation mittels Zentrifugierung entfernt. Eine derartige Phasenseparation wird jedoch nur bei strikter Einstellung der Temperaturbedingungen erreicht, wobei die Temperierung der verwendeten Zentrifuge besonders aufwendig ist. Durch die wiederholte Inkubation bei Tempteraturen von 50°C bzw. 40°C können empfindliche Moleküle irreversibel geschädigt werden.

In der EP-A-0 308 239 ist vorgeschlagen, das Endotoxin aus einer wäßrigen, aufgereinigten, biologisch brauchbare Nukleinsäure enthaltenden Lösung dadurch zu entfernen, daß der Lösung ein dialysierbares Tensid zugegeben wird und die Lösung dann in Kontakt mit einem wasserunlöslichen Endotoxinsorbens gebracht wird, um die Endotoxine an das Sorbens zu binden. Die verbleibende Flüssigphase wird anschließend von dem Sorbens getrennt und das Tensid durch Dialyse entfernt. Auf diese Weise erhält man eine tensidfreie wäßrige Lösung des biologischen Makromoleküls mit einem reduzierten Gehalt an Endotoxinen, die für die in vivo-Anwendung brauchbar ist.

Ein ähnliches Verfahren ist der WO 97/29113 zu entnehmen. Bei diesem Verfahren werden die Endotoxine nach der Aufreinigung der Nukleinsäuren in einem weiteren Schritt mittels Hydroxylapatit-Chromatographie entfernt. In beiden Fällen entsteht ein erhöhter apparativer Aufwand. Auch ist der Zeitbedarf beträchtlich.

In der WO 95/21177 und der WO 95/21179 wird neben anderen Verfahren zur Endotoxinentfernung vorgeschlagen, das filtrierte oder zentrifugierte Lysat (cleared lysate) mit einem Puffer zu versetzen, der u. a. 10 % Triton X-100® bzw. Triton X-114® enthält. Die Lösung wird bei niedrigen Temperaturen über längere Zeit vorinkubiert, bevor sie der chromatographischen Aufreinigung zugeführt wird. Diesem Verfahren haften die gleichen Nachteile an wie dem zuvor beschriebenen Verfahren. Hinzu kommt, daß bei allen Verfahren die Entfernung der Endotoxine einen zusätzlichen verfahrensschritt erfordert, der die Aufreinigungsund Isolierprozedur erheblich verlängert und kompliziert macht. Bei empfindlichen Nukleinsäuremolekülen kann dies zu Ausbeuteverlusten führen.

In der WO 99/63076 ist ein Verfahren zur Aufreinigung von Plasmid DNA beschrieben, bei dem die Aufreinigung ebenfalls an einem Anionenaustauscher geschieht. Zur Entfernung von Endotoxinen und Proteinverunreinigungen wird das Lysat vor dem Aufbringen auf die Anionenaustauschersäule mit einem nichtionischen Tensid vermischt, beispielsweise 2 % Triton X-114®. Das Tensid kann vor Aufbringen des Lysats auf die Anionenaustauschersäule mittels Inkubation bei 4°C und anschließende Pipettierung entfernt werden. Es wird jedoch auch auf die Möglichkeit hingewiesen, das mit dem Tensid versehene Lysat direkt auf die Anionenaustauschersäule zu geben. Anschließend werden die an der Säule gebundenen Endotoxine und die Reste des Tensid mittels eines Äthanolwasch-Schrittes entfernt, bevor die verbliebene Plasmid DNA eluiert wird.

Der Erfindung liegt die Aufgabe zugrunde, ein Verfahren zur Herstellung von endotoxinfreien oder endotoxinarmen biologischen Nukleinsäuren bereitzustellen, das einfach, schnell und demnach kostengünstig durchführbar und dabei sicher reproduzierbar ist. Eine weitere Aufgabe besteht darin, eine Chromatographiesäule zur Durchführung dieses Verfahrens bereitzustellen.

Diese Aufgabe wird erfindungsgemäß dadurch gelöst, daß zunächst das Tensid mit dem Anionenaustauscher in Kontakt gebracht und erst danach die biologische Lösung zwecks Aufreinigung der Nukleinsäuren/dem Anionenaustauscher zugegeben und mit dem Tensid in Kontakt gebracht wird und die Endotoxine zeitgleich mit der Adsorbtion der Nukleinsäuren an dem Anionenaustauscher aus diesem ertfernt werden. Grundgedanke der Erfindung ist es somit, die Endotoxine aus der Lösung dadurch zu entfernen, daß die Endotoxine mit den zuvor auf den Anionenaustauscher gegebenen Tensiden zeitgleich mit der chromatographischen Aufreinigung in Wechselwirkung treten und damit nicht an den Anionenaustauscher binden. Sie werden folglich aus dem Anionenaustauscher ausgewaschen, wobei der Auswaschvorgang durch den Einsatz detergenzhaltiger Lösungen unterstützt werden kann. Dabei hat sich gezeigt, daß hierdurch eine sehr wirksame Abreicherung von Endotoxinen erzielt wird, ohne daß es hierzu zeitaufwendiger Inkubations-, Extraktions- oder Waschschritte bedarf. Dadurch kommt es zu einer deutlichen Einsparung von Zeit- und Arbeisaufwand.

Unter dem Begriff "Tensid" ist auch eine Mischung verschiedener Tenside zu verstehen. Dabei sollte das Tensid zur Gruppe der Polyethylenglycolether gehören und vorzugsweise Triton X-114® sein.

Als besonders vorteilhaft hat es sich erwiesen, das nichtionosche Tensid als Bestandteil von ohnehin am chromatographischen Material angewendeten Waschpuffern zuzuführen. Alternativ, besser noch in Kombination dazu, sollte der Anionenaustauscher und/oder ein Abschlußfilter in einer dieses Material enthaltenen Chromatographiesäule mit dem Tensid imprägniert werden. Dabei sollte das Tensid in dem Waschpuffer und/oder in dem Anionenaustauscher und/oder in dem Abschlußfilter in einer Konzentration von 0,1 bis 10 % vorliegen.

Was den zweiten Teil der Aufgabe angeht, ist erfindungsgemäß vorgesehen, daß der Anionenaustauscher und/oder der eingangsseitige Abschlußfilter mit einem nichtionischen Tensid, beispielsweise Triton X-114®, imprägniert ist bzw. sind. Dabei sollte das Tensid mit einer Konzentration von 0,1 bis 10 % vorliegen. Als Anionenaustauschermaterial kann ein poröses oder nichtporöses, mit Anionenaustauscherfunktionen modifiziertes, anorganisches oder organisches Sorbens eingesetzt werden. Als Abschlußfilter kommen makroporöses, gesintertes Glas oder ein solcher Kunststoff sowie verdichtete oder miteinander verwobene synthetische Vliese aus Polyamid, Polyester, Polypropylen, Cellulose oder Kombinationen dieser Materialien in Frage. Der Abschlußfilter kann in der Dicke stark variieren. Je größer die Dicke, desto mehr Tensid kann er für den Prozeß der Endotoxinentfernung binden. Vorteilhafterweise liegt die Dicke zwischen 1 und 15 mm.

### Beispiel:

### Aufreinigung von Plasmid DNA aus E. coli

In einem entsprechenden Kulturkolben werden 100 ml LB-Medium mit einem Antibiotikum (Ampicillin®, Endkonzentra tion 100 µg/ml) versetzt und mit einer einzelnen Bakterienkolonie (E. coli Stamm DH5α, transformiert mit der Plasmid-DNA pBluescript® SK) von einer entsprechenden Agarplatte inoculiert. Die Anzucht der Bakterien erfolgt 12-14 Stunden bei 37°C in einem Schüttelinkubator. Nach dem Abzentrifugieren der Bakterienkultur wird das so erhaltene Pellet in 12 ml Resuspendierungspuffer (50 mM Tris/HCl, pH 8,0, 10 mM EDTA, 100 µg RNAse A/ml) resuspendiert, anschließend mit 12 ml Lysis Lösung (200 mM NaOH, 1 % SDS) versetzt, vorsichtig durch Umschwenken gemischt und 5 min bei Raumtemperatur inkubiert. Anschließend werden 12 ml Neutralisierungspuffer (3,0 M Kaliumacetat, pH 5,5) versetzt, die Lösung durch Umschwenken durchmischt und 5 min auf Eis inkubiert. Das neutralisierte Bakterien-Lysat wird durch Filtration mit einem Faltenfilter geklärt.

Eine Nucleobond® EF 500 Kartusche, enthaltend ein chromatographisches Material, wird mit 5 ml Äqüilibrierungspuffer (N2-EF, 100 mM Tris/Phosphat, pH 6,3, 15 % Ethanol, 900 mM KCl, 0,5 % Triton X-114® ) äquilibriert. Das durch Filtration geklärte Bakterienlysat wird auf die Kartusche aufgetragen. Anschließend werden die folgenden Waschpuffer nacheinander auf die Kartusche aufgegeben: 4 x 12 ml Puffer N3-EF (100 mM Tris/Phosphat, pH 6,3, 15 % Ethanol, 1150 mM KCl, 0,5 % Triton X-114®, 4 x 12 ml N4-EF). Die Elution der aufgereinigten Plasmid-DNA erfolgt durch Zugabe des Elutionspuffers N5-EF (100 mM Tris/Phosphat, pH 7,0, 15 % Ethanol, 1500 mM KCl). Die eluierte DNA wird durch Zugabe von 0,7-0,8 % Isopropanol präzipitiert. Nach einer Zentrifugation (30 min>15000 x g) wird das DNA-Pellet nochmals mit endotoxinfreiem, auf 4°C vorgekühltem 70 % Ethanol gewaschen. Nach einem Trockenschritt wird die DNA in endotoxinfreiem TE-Puffer resuspendiert.

## Patentansprüche

1. Verfahren zur Aufreinigung von Nukleinsäuren aus Ausgangsmaterialen, bei dem die Nukleinsäuren, enthalten in einer biologischen Lösung, an einem Anionenaustauscher aufgereinigt werden und bei dem mit Hilfe eines nichtionischen Tensids Endotoxine teilweise oder ganz entfernt werden, **dadurch gekennzeichnet, daß** zunächst das Tensid mit dem Anionenaustauscher in Kontakt gebracht und erst danach die biologische Lösung zwecks Aufreinigung der Nukleinsäuren dem Anionenaustauscher zugegeben und mit dem Tensid in Kontakt gebracht wird und die Endotxine zeitgleich mit der Adsorption der Nukleinsäuren an dem Anionenaustauscher aus diesem entfernt werden.

2. Verfahren nach Anspruch 1, **dadurch gekennzeichnet, daß** das Tensid zur Gruppe der Polyethylenglycolether gehört.

3. Verfahren nach Anspruch 1 oder 2, **dadurch gekennzeichnet, daß** als Tensid Triton X-114® verwendet wird.

4. Verfahren nach einem der Ansprüche 1 bis 3, **dadurch gekennzeichnet, daß** das Tensid als Bestandteil eines oder mehrerer Waschpuffer verwendet wird.

5. Verfahren nach einem der Ansprüche 1 bis 4, **dadurch gekennzeichnet, daß** der Anionenaustauscher und/oder ein eingangsseitiger Abschlußfilter in einer dieses Material enthaltenden Chromatographiesäule mit dem Tensid imprägniert wird.

6. Verfahren nach einem der Ansprüche 1 bis 5, **dadurch gekennzeichnet, daß** das Tensid in dem bzw. den waschpuffer(n) und/oder in dem Anionenaustauscher und/oder in dem Abschlußfilter in einer Konzentration von 0,1 bis 10 % vorliegt.

7. Chromatographiesäule zur Durchführung des Verfahrens nach einem der Ansprüche 1 bis 6, die ein von Abschlußfiltern eingeschlossenen Anionenaustauscher enthält, **dadurch gekennzeichnet, daß** der Anionenaustauscher und/oder der eingangsseitige Abschlußfilter mit einem nichtionischen Tensid imprägniert ist bzw. sind.

8. Chromatographiesäule nach Anspruch 7, **dadurch gekennzeichnet, daß** das Tensid zur Gruppe der Polyethylenglycolether gehört.

9. Chromatographiesäule nach Anspruch 7 oder 8, **dadurch gekennzeichnet, daß** das Tensid Triton X-114® ist.

10. Chromatographiesäule nach einem der Ansprüche 7 bis 9, **dadurch gekennzeichnet, daß** das Tensid in dem Abschlußfilter und/oder in dem Anionenaustauscher in einer Konzentration von 0,1 bis 10 % vorliegt.

11. Chromatographiesäule nach einem der Ansprüche 8 bis 10, **dadurch gekennzeichnet, daß** das Abschlußfilter eine Dicke von 1 bis 15 mm hat.

## Claims

1. Process for the purification of nucleic acids from starting materials, in which the nucleic acids, which are contained in a biological solution, are purified in an anion exchanger and partly or completely removed using a non ionic endotoxin surfactant, **characterised by** the fact that first the surfactant is put in contact with an anion exchanger and then the biological solution is added to the anion exchanger in order to purify the nucleic acids and the endotoxins are removed from this simultaneously with the adsorption of nucleic acids in the anion exchanger.

2. Process according to claim 1, **characterised by the fact** that the surfactant belongs to the polyethylene glycol ether group.

3. Process according to claim 1 or 2, **characterised by the fact** that the surfactant Triton X-114 ® is used.

4. Process according to one of claims 1 to 3, **characterised by the fact** that the surfactant is used as a component of one or several washing buffers.

5. Process according to one of claims 1 to 4, **characterised by the fact** that the anion exchanger and/or a post filter on the inlet side in a chromatography column containing this material is impregnated with surfactant.

6. Process according to one of claims 1 to 5, **characterised by** the fact that the surfactant is in the washing buffer(s) and/or in the anion exchanger and/or in the post filter in a concentration of 0.1 to 10%.

7. Chromotography column for carrying out the process according to one of claims 1 to 6, which contains an anion exchanger enclosed by post filters, **characterised by the fact** that the anion exchanger and/or the post filter on the inlet side is/are impregnated with a non ionic surfactant.

8. Chromotography column according to claim 7, **characterised by the** fact that the surfactant belongs to the polyethylene glycol ether group.

9. Chromotography column according to claim 7 or 8, **characterised by the fact** that the surfactant Triton X-114® is used.

10. Chromotography column according to one of claims 7 to 9, **characterised by** the fact that the surfactant is present in the post filter and/or in the anion exchanger in a concentration of 0.1 to 10%.

11. Chromotography column according to one of claims 8 to 10, **characterised by the fact** that the post filter is 1 to 15 mm thick.

## Revendications

1. Procédé de purification d'acides nucléiques à partir de matériaux de départ, dans lequel les acides nucléiques, contenus dans une solution biologique, sont purifiés au niveau d'un échangeur anionique, et dans lequel, à l'aide d'un tensioactif non ionique, on élimine partiellement ou totalement les endotoxines, **caractérisé en ce que** le tensioactif est d'abord mis en contact avec l'échangeur anionique, et ce n'est qu'après que la solution biologique est ajoutée à l'échangeur anionique et est mise en contact avec le tensioactif à des fins de purification des acides nucléiques, et les endotoxines sont éliminées de ceux-ci simultanément à l'adsorption des acides nucléiques au niveau de l'échangeur anionique.

2. Procédé selon la revendication 1, **caractérisé en ce que** le tensioactif appartient au groupe des polyéthylèneglycoléthers.

3. Procédé selon la revendication 1 ou 2, **caractérisé en ce que** l'on utilise comme tensioactif le Triton X-114®.

4. Procédé selon l'une des revendications 1 à 3, **caractérisé en ce que** l'on utilise le tensioactif comme composant d'un ou de plusieurs tampon(s) de lavage.

5. Procédé selon l'une des revendications 1 à 4, **caractérisé en ce que** l'échangeur anionique et/ou un filtre d'élimination côté entrée est imprégné du tensioactif dans une colonne de chromatographie contenant ce matériau.

6. Procédé selon l'une des revendications 1 à 5, **caractérisé en ce que** le tensioactif est présent dans le et/ou les tampon(s) de lavage et/ou dans l'échangeur anionique et/ou dans le filtre d'élimination selon une concentration comprise entre 0,1 et 10 %.

7. Colonne de chromatographie permettant d'exécuter le procédé selon l'une des revendications 1 à 6, qui contient un échangeur anionique enfermé par des filtres d'élimination, **caractérisée en ce que** l'échangeur anionique et/ou le filtre d'élimination côté entrée est ou sont imprégné(s) d'un tensioactif non ionique.

8. Colonne de chromatographie selon la revendication 7, **caractérisée en ce que** le tensioactif appartient au groupe des polyéthylèneglycoléthers.

9. Colonne de chromatographie selon la revendication 7 ou 8, **caractérisée en ce que** l'on utilise comme tensioactif le Triton X-114®.

10. Colonne de chromatographie selon l'une des revendications 7 à 9, **caractérisée en ce que** le tensioactif est présent dans le filtre d'élimination et/ou dans l'échangeur anionique selon une concentration comprise entre 0,1 et 10 %.

11. Colonne de chromatographie selon l'une des revendications 8 à 10, **caractérisée en ce que** le filtre d'élimination possède une épaisseur comprise entre 1 et 15 mm.
